# EUROPEAN PATENT SPECIFICATION

(11) **EP 1 183 224 B1**
(45) Date of publication and mention of the grant of the patent: **04.08.2004**
(21) Application number: 01903473.5
(22) Date of filing: 01.02.2001
(51) Int. Cl.: C07C 41/16, C07C 43/20

(54) **PROCESS FOR PREPARING ALKOXY OR ARYLMETHOXY AROXYETHANES**
VERFAHREN ZUR HERSTELLUNG VON ALKOXY- ODER ARYLMETHOXY-AROXYETHANEN
PROCEDE DE PREPARATION D'AROXYETHANES ALCOXY OU ARYLMETHOXY

(30) Priority: 17.02.2000 US 183275 P
(43) Date of publication of application: 06.03.2002
(73) Proprietor: Appleton Papers Inc., Appleton, WI 54912-0359 (US)
(72) Inventor: MATHIAPARANAM, Ponnampalam, Appleton, WI 54915-7203 (US); BERGGREN, Debra Arlene, Kimberly, WI 54136 (US)
(74) Representative: Farwell, William Robert
(86) International application number: PCT/US2001/003328
(87) International publication number: WO 2001/060775

(56) References cited:
- EP-A- 0 484 874
- US-A- 2 987 555
- US-A- 4 341 905
- US-A- 5 179 068

## Description

### BACKGROUND OF THE INVENTION

### 1. Field of the Invention.

This invention relates to alkoxy or arylmethoxy ethanes. This invention particularly concerns a process for preparation of such compounds. More particularly, the invention teaches a novel process for preparation of 2-alkoxy (or 2-arylmethoxy)-1-aroxyethanes, a class of compounds useful in a variety of diverse applications such as improved sensitizers or modifiers for thermal sensitive papers and as dispersants, emollients, and texture enhancing agents in cosmetics and lotions.

### 2. Description of Related Art.

There are several methods described in the literature for preparing 1-benzyloxy-2-phenoxyethane represented by the structure A:

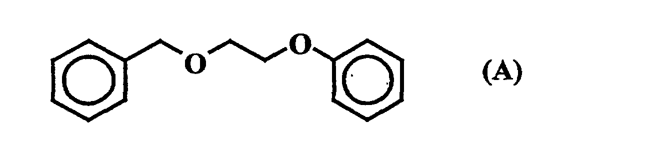

C. Berggardh [Finska Kemistsamf.Medd., 42, 76 (1933)] and E.M. Van Duzee and H. Atkins [J. Amer. Chem. Soc., 57, 147 (1935)] prepared (A) by reacting sodium 2-phenoxyethoxide with benzyl chloride. Also, C.L. Butler and A.G. Renfrew [J. Amer. Chem. Soc., 60, 1582 (1938)] and C.L. Butler and L.H. Cretcher [U.S. Patent, 2,172,606 (1939)] obtained (A) by treating 2-benzyloxyethyl p-toluenesulfonate with potassium phenoxide. These two methods require the preparation of one or both starting materials in a separate step and involves the use of either potassium or sodium metal that are expensive and difficult to handle in scale up operations. J.S. Bradshaw, B.A. Jones and J.S. Gebhard [J. Org. Chem., 48, 1127 (1983)] made (A) by reductive desulfurization of 2-phenoxyethyl thiobenzoate using Raney nickel. Again, the starting material thiobenzoate, prepared from not readily available 2-phenoxyethyl benzoate by thionation, makes this process not amenable to scale up. A. Goto [U.S. Patent No. 5,179,068] described a method for preparing 1,4-bis (2-aroxyethoxymethyl) benzenes by reacting 2-phenoxyethanol with p-xylylene dichloride and aqueous sodium hydroxide using trioctylmethylammonium chloride as catalyst in toluene. Goto also, described a process for making 1,4-bis (2-aroxyethoxymethyl) benzenes in two steps starting from substituted phenol and ethylene carbonate. In the first step, the substituted phenol and ethylene carbonate were heated with catalytic amounts of potassium carbonate in chlorobenzene to generate the corresponding substituted phenoxyethanol. In the second step, the substituted phenoxyethanol was reacted with p-xylylene chloride and aqueous sodium hydroxide using trioctylmethylammonium chloride as catalyst in chlorobenzene. The success of this tandem two step process depends on the complete conversation of the substituted phenol to the corresponding substituted phenoxyethanol in the first step; otherwise, a mixture of inseparable products are formed, resulting in low yield of the desired product.

### DETAILED DESCRIPTION

The present invention is a novel process for manufacturing 2-alkoxy (or 2-arylmethoxy)-1-aroxyethanes using a one-pot, two-step procedure. The novel process comprises reacting substituted or unsubstituted phenol (or naphthol) with ethylene carbonate in the presence of a first catalyst, with or without solvent, and reacting the product formed in the first step with alkyl or aralkyl halide (sulfate or sulfonate) and metal hydroxide in the presence of a second catalyst with or without solvent.

This invention teaches a process for preparing 2-alkoxy(or 2-arylmethoxy)-1-aroxyethanes. Particularly, this invention teaches a novel process for preparing 2-alkoxy (or 2-arylmethoxy)-1-aroxy-ethanes represented by the formula (I): 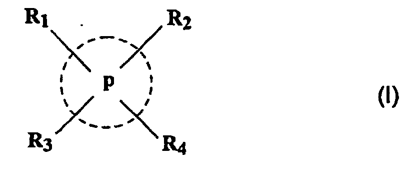 wherein P is selected from phenyl and naphthyl moieties.

Formula (IIA) depicts when P in the structure of Formula 1 is replaced by phenyl

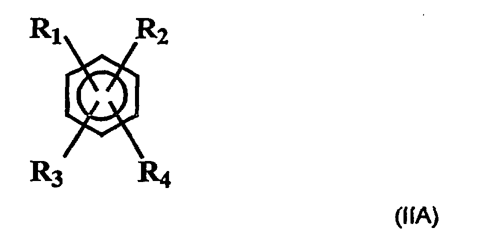

Formula IIB depicts when P in the structure of Formula I is replaced by naphthyl.

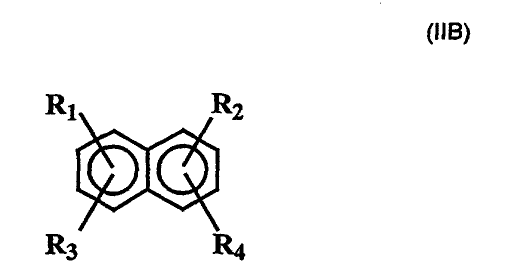

In each of formulas I, IIA and IIB the substituents R₁, R₂ and R₃ are independently hydrogen, alkyl, alkoxy, aryl, aralkyl, aralkoxy, halogen, alkoxyalkoxy and aralkoxyalkoxy; and R₄, is independently alkoxyethyl, alkoxyethoxy and aralkoxyethoxy.

In the substituents R₁, R₂, R₃ and R₄, the alkyl moieties in alkyl, alkoxy, aralkyl, aralkoxy, alkoxyalkyl, alkoxyalkoxy and aralkoxyalkoxy groups contain one through eight carbon atoms.

This invention teaches an improved process for manufacturing 2-alkoxy (or 2-arylmethoxy)-1-aroxyethanes (VIII) using a one-pot, two-step procedure from readily available materials. The process of the invention is diagrammed as follows:

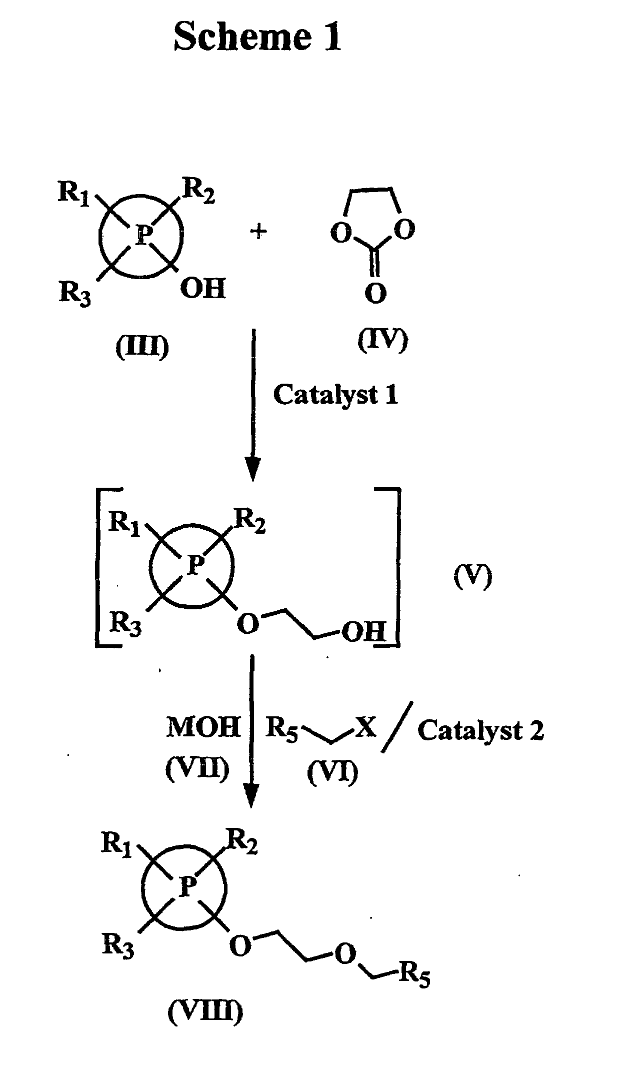

The substituents P, R₁, R₂ and R₃ are as defined previously. R₅ is either a substituted or unsubstituted phenyl or naphthyl group. The substituents on the phenyl or naphthyl groups include alkyl (C₁-C₈), alkoxy (C₁-C₈), aroxy, aralkoxy (C₁-C₈ alkyl) and halogen. For clarity "aralkoxy (C₁- C₈)" herein will refer to the alkyl moiety as having from one to eight carbons.

The process comprises reacting substituted or unsubstituted phenols (or naphthols) (III) with ethylene carbonate (IV) using catalyst 1 without a solvent and reacting the product formed (V) in the first step with alkyl or aralkyl halide (sulfate or sulfonate) (VI) and metal hydroxide in the presence of catalyst 2 with or without a solvent.

By heating the phenol (or naphthol) (III) with slight excess of ethylene carbonate and the catalyst 1 without solvent, the phenol (or naphthol) is completely converted to the corresponding 2-phenoxy (or 2-naphthoxy) ethanol (V). The reaction temperature may be selected from 50°C to 200°C depending on the phenol (or naphthol) used. Most of the phenols (or naphthols) react in the preferred temperature range from 140°C to 160°C.

The catalyst 1 that is suitable for this reaction include metal halides, quartemary ammonium halides and quartemary phosphonium halides. Preferred catalysts include sodium chloride, sodium bromide, sodium iodide, potassium chloride, potassium bromide, potassium iodide, tetraethylammonium chloride, tetraethylammonium bromide, tetraethylammoniumiodide, tetrabutylammonium chloride, tetrabutylammonium bromide, tetrabutylammonium iodide, methyltrioctylammonium chloride (aliquat 336), tetraethylphosphonium chloride, tetraethylphosphonium bromide and tetraethylphosphonium iodide. Also, a combination of quarternary ammonium salt or quarternary phosphonium salt other than halides and metal halides can be used as catalyst 1.

The intermediate (V) was then mixed with alkyl or aralkyl halide (sulfate or sulfonate) (VI), metal hydroxide (VII) and catalyst 2, heated and stirred vigorously. Powdered metal hydroxide was used in the solvent free procedure. Aqueous solution (40-50%) of metal hydroxide was used with a solvent in the solvent procedure. Preferred metal hydroxides include sodium hydroxide and potassium hydroxide and the preferred solvents are aliphatic or aromatic hydrocarbons or chlorohydrocarbons. Catalyst 2 may be either quartemary ammonium salt or quarternary phosophonium salt. Preferred catalyst 2 are tetrabutylammonium hydrogen sulfate, tetrabutylammonium halide, tetraethylammonium halide, methyltrioctylammonium choride (also known as aliquat 336) and tetraethylphosphonium halide. The reaction temperature for the second step is dependent on the solvent used. The preferred temperature range is room temperature to 55°C for low boiling point solvents and 50-100°C for high boiling point solvents. For the solvent free procedure 90-100°C temperature range is preferred.

By carrying out the step 1 of this process in excess of ethylene carbonate (IV), the phenol (or naphthol) (III) is completely converted to the corresponding 2-phenoxy (or 2-naphthoxy) ethanol (V). No solvent need be used (solvent being optional but preferably omitted in Step 1) and excess ethylene carbonate and lower boiling materials are removed under reduced pressure. This complete conversion is important; otherwise, a mixture of unwanted by products are obtained by reaction of (III) with (VI). This is one of the features of this tandem process.

In step 2, (V) is converted to (VIII) using either a solvent-free or a solvent procedure. In the solvent-free procedure, a solid liquid phase transfer catalysis reaction was selected because it gives complete conversion. Metal hydroxides should be finely powdered and the stirring should be vigorous to produce efficient conversion. In the solvent procedure, an aqueous solution of the metal hydroxide and a suitable solvent was used as in traditional phase transfer catalysis reaction. Here again, vigorous stirring is recommended for optimum conversion.

The solvent-free options and the one-pot process enable scale up for commercial production. Furthermore, by changing the phenol (or naphthol) (III) and alkyl or aralkyl halide (sulfate or sulfonate) (VI), a series of 2-alkoxy (2-arylmethoxy)-1-aryloxyethanes (VIII) were prepared. Some of compounds (VIII) prepared are listed below:

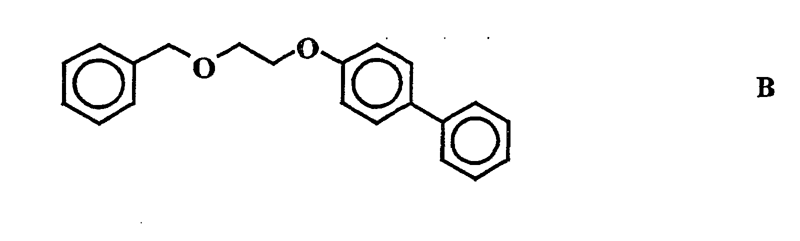

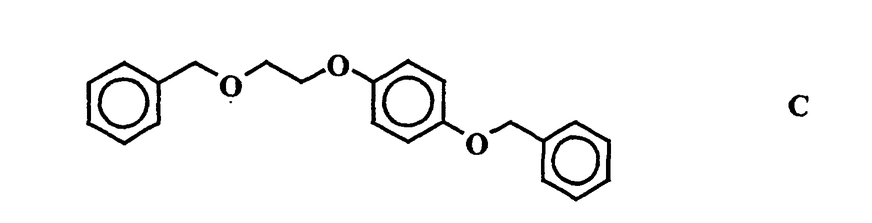

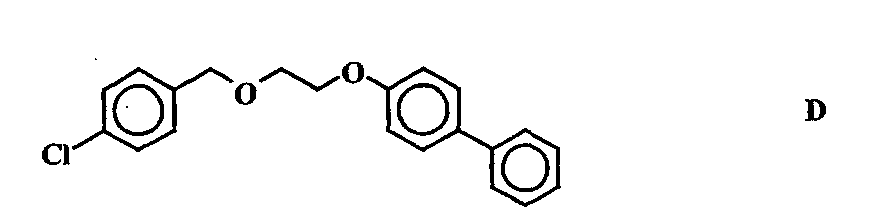

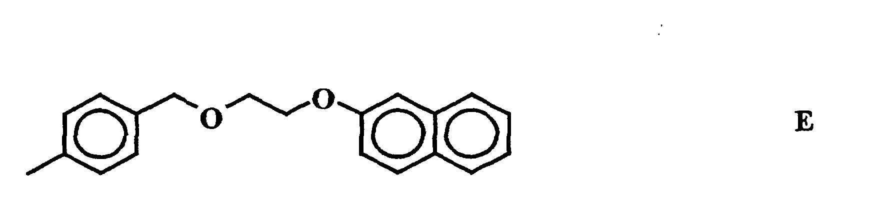

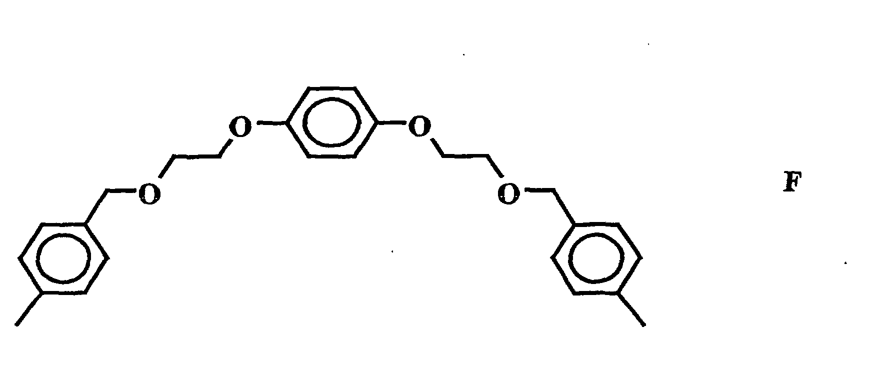

### DESCRIPTION OF THE PREFERRED EMBODIMENTS

In the following examples, general procedures for preparing certain compounds listed above are described; the examples are not intended to be exhaustive and the moieties, as previously defined, are all eligible for use in any combination in preparing the compounds. Unless otherwise noted, all measurements, percentages and parts are by weight and in the metric system.

### EXAMPLE 1

### Preparation of 1-Benzyloxy-2-[4-(benzyloxy)phenoxy]ethane (Compound C) by Solvent-Free Reaction.

4-Benzyloxyphenol (60.6g, 0.3 mole), ethylene carbonate (32.0g, 0.36 mole) and sodium chloride (4.0g, 0.07 mole) were placed in a 500 ml, three-necked, round-bottom flask equipped with a mechanical stirrer and a reflux condenser. The reaction mixture was stirred and heated to 155°C. After 9 hours, the reaction mixture was cooled to 100°C and most of the lower boiling materials were removed under reduced pressure. Then, tetrabutylammonium hydrogen sulfate (4.0g, 0.012 mole) and finely powdered potassium hydroxide (28.0g, 0.5 mole) were added to the reaction mixture and the vigorous stirring was continued. After five minutes, benzyl chloride (45.0g, 0.36 mole) was added slowly with vigorous stirring. After four hours, toluene (200 ml) and water (60 ml) were added and stirring was continued for another ten minutes. The toluene layer was separated and the aqueous layer was extracted twice with toluene. The toluene layers were combined and washed with water, dried and concentrated. The residue was recrystallized from methanol. Yield: 82.6g (82%), White solid, M.P.: 71-73°C.

### EXAMPLE 2

### Preparation of 1-Benzyloxy-2-[4-(benzyloxy)phenoxy]ethane (Compound C)

4-Benzyloxyphenol (60.6g, 0.3 mole), ethylene carbonate (32.0g, 0.36 mole) and sodium iodide (2.25g, 0.015 mole) were placed in a 500 ml, three-necked, round-bottom flask equipped with a mechanical stirrer and a reflux condenser. The reaction mixture was stirred and heated at 155°C. After 5 hours, the reaction mixture was cooled to 110°C and most of the lower boiling materials were removed under reduced pressure. Then, tetrabutylammonium hydrogen sulfate (4.0g, 0.12 mole), benzyl chloride (45.0g, 0.35 mole), toluene (200 ml) and sodium hydroxide (20.0g, 0.5 mole / 40 ml of water) were added to the reaction mixture and the vigorous stirring was continued while the reaction mixture temperature was lowered to 90°C. After overnight at this temperature, the reaction mixture was cooled to room temperature and transferred to a separatory funnel. The toluene layer was separated and the aqueous layer was extracted twice with toluene. Toluene extracts were combined, washed with water, dried, treated with Norit A and filtered. The filtrated was passed through a short column of silica gel and eluted with toluene. Fractions containing the product were collected, combined and concentrated. The residue was recrystallized from methanol. Yield: 72.6g (72%), White solid, M.P.: 71-73°C.

### EXAMPLE 3

### Preparation of 1-(4-Chlorobenzyloxy)-2-(4-phenylphenoxy)ethane (Compound D) by Solvent-Free Reaction

4-Phenylphenol (34.0g, 0.2 mole), ethylene carbonate (20.0g, 0.227 mole) and tetrabutylammonium bromide (6.5g, 0.02 mole) were heated to 155°C with stirring in a three-necked, round-bottom flask equipped with a mechanical stirrer and a reflux condenser. After 5 hours, the reaction mixture was cooled to 110°C, most of the lower boiling materials were removed under reduced pressure. Finely powdered potassium hydroxide (17.0g, 0.3 mole) was added and the reaction mixture was stirred for 5 minutes. Then, 4-chloro-benzyl chloride (35.0g, 0.217 mole) was added and the reaction was kept at 110°C with vigorous stirring. After 4 hours, toluene (250 ml) was added and the reaction mixture was cooled to 60°C. Water (100 ml) was added and stirring continued. Toluene layer was separated and the aqueous layer was extracted twice with warm toluene. The toluene extracts were combined, washed with water, dried and concentrated. The residue was recrystallized from toluene/methanol. Yield: 55.8g (82%), white solid, M.P.: 85-87°C.

## Claims

1. A novel process for manufacture of compounds of the formula 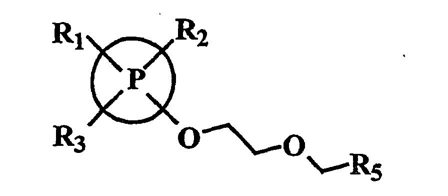 wherein P is selected from phenyl and naphthyl;
wherein R₁, R₂ and R₃ are each independently selected from the group consisting of hydrogen, alkyl, alkoxy, aryl, aralkyl, aralkoxy, halogen, alkoxyalkoxy, and aralkoxyalkoxy;
wherein R₅ is selected from substituted or unsubstituted phenyl, and substituted or unsubstituted naphthyl, the substituents being each independently selected from alkyl (C₁-C₈), alkoxy (C₁-C₈), aroxy, aralkoxy and halogen;
the process comprising reacting substituted or unsubstituted phenols or naphthols of the formula 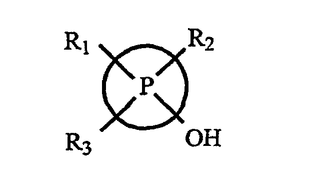 with ethylene carbonate in the presence of a first catalyst selected from the group consisting of metal halide, quarternary ammonium halide and quartemary phosphonium halide thereby forming an intermediate of the formula 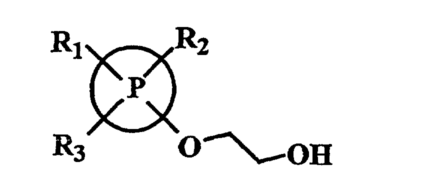 reacting the intermediate with a first compound selected from the group consisting of alkyl halide, aralkyl halide, alkyl sulfate, aralkyl sulfate, alkyl sulfonate and aralkyl sulfonate together with a metal hydroxide in the presence of a second catalyst, the second catalyst selected from quarternary ammonium salt or quartemary phosphonium salt.

2. The process according to claim 1 wherein the first catalyst is selected from the group consisting of sodium chloride, sodium bromide, sodium iodide, potassium chloride, potassium bromide, potassium iodide, tetraethylammonium chloride, tetraethylammonium bromide, tetraethylammoniumiodide, tetrabutylammonium,chloride, tetrabutylammonium bromide, tetrabutylammonium iodide, methyltrioctylammonium chloride, tetraethylphosphonium bromide, and tetraethylphosphonium iodide.

3. The process according to claim 1 wherein the second catalyst is selected from the group consisting of tetrabutylammonium hydrogen sulfate, tetrabutylammonium halide, tetraethylammonium halide, methyltrioctylammonium chloride, and tetraethylphosphonium halide.

4. The process according to claim 1 wherein the reaction of the intermediate is carried out in a solvent selected from the group consisting of aliphatic hydrocarbons, aromatic hydrocarbons and chlorohydrocarbons.

5. A novel process according to claim 1 for manufacture of compounds of the formula 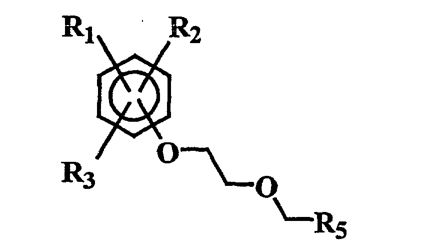 wherein R₁, R₂ and R₃ are each independently selected from the group consisting of hydrogen, alkyl, alkoxy, aryl, aralkyl, aralkoxy, halogen, alkoxyalkoxy, and aralkoxyalkoxy;
wherein R₅ is selected from substituted or unsubstituted phenyl, the substituents being each independently selected from alkyl (C₁-C₈), alkoxy (C₁-C₈), aroxy, aralkoxy and halogen;
the process comprising reacting substituted or unsubstituted phenols of the formula 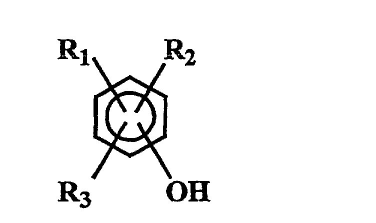 with ethylene carbonate in the presence of a first catalyst selected from the group consisting of metal halide, quaternary ammonium halide and quartemary phosphonium halide thereby forming an intermediate of the formula 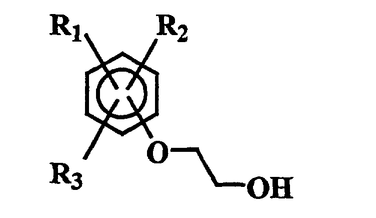 reacting the intermediate with a first compound selected from the group consisting of alkyl halide, aralkyl halide, alkyl sulfate, aralkyl sulfate, alkyl sulfonate and aralkyl sulfonate together with a metal hydroxide in the presence of a second catalyst, the second catalyst selected from quartemary ammonium salt or quartemary phosphonium salt.

6. The process according to claim 5 wherein the first catalyst is selected from the group consisting of sodium chloride, sodium bromide, sodium iodide, potassium chloride, potassium bromide, potassium iodide, tetraethylammonium chloride, tetraethylammonium bromide, tetraethylammoniumiodide, tetrabutylammonium chloride, tetrabutylammonium bromide, tetrabutylammonium iodide, methyltrioctylammonium chloride, tetraethylphosphonium bromide, and tetraethylphosphonium iodide.

7. The process according to claim 5 wherein the second catalyst is selected from the group consisting of tetrabutylammonium hydrogen sulfate, tetrabutylammonium halide, tetraethylammonium halide, methyltrioctylammonium chloride and tetraethylphosphonium halide.

8. The process according to claim 5 wherein the reaction of the intermediate is carried out in a solvent selected from the group consisting of aliphatic hydrocarbons, aromatic hydrocarbons and chlorohydrocarbons.

9. A novel process according to claim 1 for manufacture of compounds of the formula 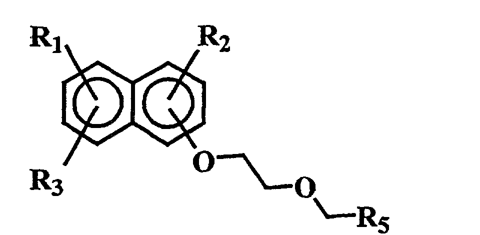 wherein R₁, R₂ and R₃ are each independently selected from the group consisting of hydrogen, alkyl, alkoxy, aryl, aralkyl, aralkoxy, halogen, alkoxyalkoxy, and aralkoxyalkoxy;
wherein R₅ is selected from substituted or unsubstituted naphthyl, the substituents being each independently selected from alkyl (C₁-C₈), alkoxy (C₁-C₈), aroxy, aralkoxy (C₁-C₈) and halogen;
the process comprising reacting substituted or unsubstituted naphthols of the formula 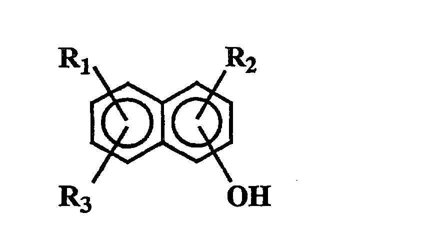 with ethylene carbonate in the presence of a first catalyst selected from the group consissting of metal halide, quartemary ammonium halide and quarternary phosphonium halide, thereby forming an intermediate of the formula 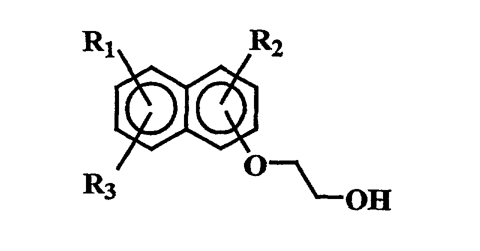 reacting the intermediate with a first compound selected from the group consisting of alkyl halide, aralkyl halide, alkyl sulfate, aralkyl sulfate, alkyl sulfonate and aralkyl sulfonate together with a metal hydroxide in the presence of a second catalyst, the second catalyst selected from quartemary ammonium salt or quartemary phosphonium salt.

10. The process according to claim 9 wherein the first catalyst is selected from the group consisting of sodium chloride, sodium bromide, sodium iodide, potassium chloride, potassium bromide, potassium iodide, tetraethylammonium chloride, tetraethylammonium bromide, tetraethylammoniumiodide, tetrabutylammonium chloride, tetrabutylammonium bromide, tetrabutylammonium iodide, methyltrioctylammonium chloride, tetraethylphosphonium bromide, and tetraethylphosphonium iodide.

11. The process according to claim 9 wherein the second catalyst is selected from the group consisting of tetrabutylammonium hydrogen sulfate, tetrabutylammonium halide, tetraethylammonium halide, methyltrioctylammonium chloride and tetraethylphosphonium halide.

12. The process according to claim 9 wherein the reaction of the intermediate is carried out in a solvent selected from the group consisting of aliphatic hydrocarbons, aromatic hydrocarbons and chlorohydrocarbons.

## Patentansprüche

1. Neues Verfahren zur Herstellung von Verbindungen der Formel: 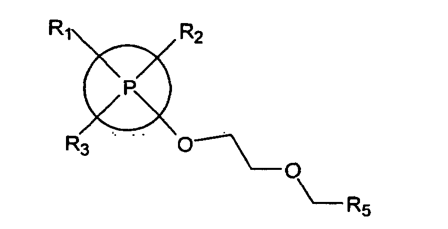 worin P ausgewählt ist aus Phenyl und Naphthyl;
R₁, R₂ und R₃ sind jeweils unabhängig voneinander ausgewählt aus Wasserstoff, Alkyl, Alkoxy, Aryl, Aralkyl, Aralkoxy, Halogen, Alkoxyalkoxy und Aralkoxyalkoxy;
R₅ ist ausgewählt aus substituiertem oder unsubstituiertem Phenyl und substituiertem oder unsubstituiertem Naphthyl, wobei die Substituenten jeweils unabhängig voneinander ausgewählt sind aus C₁₋₈-Alkyl, C₁₋₈-Alkoxy, Aryloxy, Aralkyloxy und Halogen;
das Verfahren umfaßt die Umsetzung substituierter oder unsubstituierter Phenole oder Naphthole der Formel: 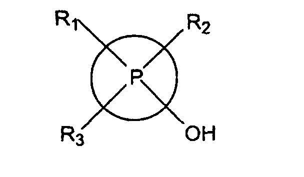 mit Ethylencarbonat in Gegenwart eines ersten Katalysators, ausgewählt aus Metallhalogenid, quaternärem Ammoniumhalogenid und quaternärem Phosphoniumhalogenid, wodurch ein Intermediat der folgenden Formel gebildet wird: 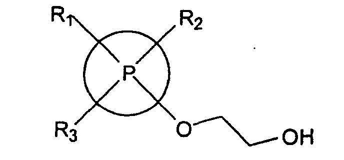 Umsetzen des Intermediats mit einer ersten Verbindung, ausgewählt aus Alkylhalogenid, Aralkylhalogenid, Alkylsulfat, Aralkylsulfat, Alkylsulfonat und Aralkylsulfonat, zusammen mit einem Metallhydroxid in Gegenwart eines zweiten Katalysators, der ausgewählt ist aus quaternärem Ammoniumsalz oder quaternärem Phosphoniumsalz.

2. Verfahren gemäß Anspruch 1, worin der erste Katalysator ausgewählt ist aus Natriumchlorid, Natriumbromid, Natriumiodid, Kaliumchlorid, Kaliumbromid, Kaliumiodid, Tetraethylammoniumchlorid, Tetraethylammoniumbromid, Tetraethylammoniumiodid, Tetrabutylammoniumchlorid, Tetrabutylammoniumbromid, Tetrabutylammoniumiodid, Methyltrioctylammoniumchlorid, Tetraethylphosphoniumbromid und Tetraethylphosphoniumiodid.

3. Verfahren gemäß Anspruch 1, worin der zweite Katalysator ausgewählt ist aus Tetrabutylammoniumhydrogensulfat, Tetrabutylammoniumhalogenid, Tetraethylammoniumhalogenid, Methyltrioctylammoniumchlorid und Tetraethylphosphoniumhalogenid.

4. Verfahren gemäß Anspruch 1, worin die Reaktion des Intermediats in einem Lösungsmittel durchgeführt wird, das ausgewählt ist aus aliphatischen Kohlenwasserstoffen, aromatischen Kohlenwasserstoffen und Chlorkohlenwasserstoffen.

5. Neues Verfahren gemäß Anspruch 1 zur Herstellung von Verbindungen der Formel: 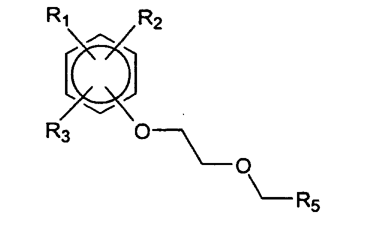 worin R₁, R₂ und R₃ jeweils unabhängig voneinander ausgewählt sind aus Wasserstoff, Alkyl, Alkoxy, Aryl, Aralkyl, Aralkoxy, Halogen, Alkoxyalkoxy und Aralkoxyalkoxy;
R₅ ist ausgewählt aus substituiertem oder unsubstituiertem Phenyl, wobei die Substituenten jeweils unabhängig voneinander ausgewählt sind aus C₁₋₈-Alkyl, C₁₋₈-Alkoxy, Aryloxy, Aralkyloxy und Halogen;
das Verfahren umfaßt die Umsetzung substituierter oder unsubstituierter Phenole der Formel: 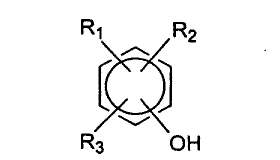 mit Ethylencarbonat in Gegenwart eines ersten Katalysators, der ausgewählt ist aus Metallhalogenid, quaternärem Ammoniumhalogenid und quaternärem Phosphoniumhalogenid, wodurch ein Intermediat der folgenden Formel gebildet wird: 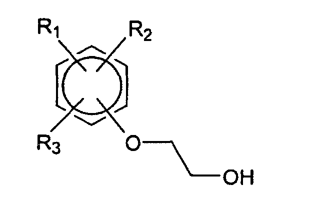 und Umsetzen des Intermediats mit einer ersten Verbindung, die ausgewählt ist aus Alkylhalogenid, Aralkylhalogenid, Alkylsulfat, Aralkylsulfat, Alkylsulfonat und Aralkylsulfonat, zusammen mit einem Metallhydroxid in Gegenwart eines zweiten Katalysators, der ausgewählt ist aus quaternärem Ammoniumsalz oder quaternärem Phosphoniumsalz.

6. Verfahren gemäß Anspruch 5, worin der erste Katalysator ausgewählt ist aus Natriumchlorid, Natriumbromid, Natriumiodid, Kaliumchlorid, Kaliumbromid, Kaliumiodid, Tetraethylammoniumchlorid, Tetraethylammoniumbromid, Tetraethylammoniumiodid, Tetrabutylammoniumchlorid, Tetrabutylammoniumbromid, Tetrabutylammoniumiodid, Methyltrioctylammoniumchlorid, Tetraethylphosphoniumbromid und Tetraethylphosphoniumiodid.

7. Verfahren gemäß Anspruch 5, worin der zweite Katalysator ausgewählt ist aus Tetrabutylammoniumhydrogensulfat, Tetrabutylammoniumhalogenid, Tetraethylammoniumhalogenid, Methyltrioctylammoniumchlorid und Tetraethylphosphoniumhalogenid.

8. Verfahren gemäß Anspruch 5, worin die Reaktion des Intermediats in einem Lösungsmittel durchgeführt wird, das ausgewählt ist aus aliphatischen Kohlenwasserstoffen, aromatischen Kohlenwasserstoffen und Chlorkohlenwasserstoffen.

9. Neues Verfahren gemäß Anspruch 1 zur Herstellung von Verbindungen der Formel 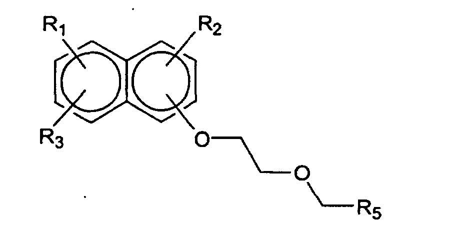 worin R₁, R₂ und R₃ jeweils unabhängig voneinander ausgewählt sind aus Wasserstoff, Alkyl, Alkoxy, Aryl, Aralkyl, Aralkoxy, Halogen, Alkoxyalkoxy und Aralkoxyalkoxy;
R₅ ist ausgewählt aus substituiertem oder unsubstituiertem Naphthyl, wobei die Substituenten jeweils unabhängig voneinander ausgewählt sind aus C₁₋₈-Alkyl, C₁₋₈-Alkoxy, Aryloxy, C₁₋₈-Aralkyloxy und Halogen;
das Verfahren umfaßt die Umsetzung substituierter oder unsubstituierter Naphthole der Formel: 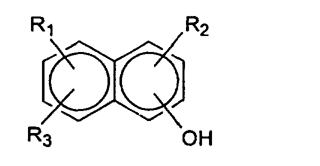 mit Ethylencarbonat in Gegenwart eines ersten Katalysators, der ausgewählt ist aus Metallhalogenid, quaternärem Ammoniumhalogenid und quaternärem Phosphoniumhalogenid, wodurch ein Intermediat der folgenden Formel gebildet wird: 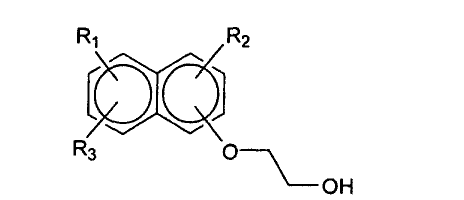 und Umsetzen des Intermediats mit einer ersten Verbindung, die ausgewählt ist aus Alkylhalogenid, Aralkylhalogenid, Alkylsulfat, Aralkylsulfat, Alkylsulfonat und Aralkylsulfonat, zusammen mit einem Metallhydroxid in Gegenwart eines zweiten Katalysators, der ausgewählt ist aus quaternärem Ammoniumsalz oder quaternärem Phosphoniumsalz.

10. Verfahren gemäß Anspruch 9, worin der erste Katalysator ausgewählt ist aus Natriumchlorid, Natriumbromid, Natriumiodid, Kaliumchlorid, Kaliumbromid, Kaliumiodid, Tetraethylammoniumchlorid, Tetraethylammoniumbromid, Tetraethylammoniumiodid, Tetrabutylammoniumchlorid, Tetrabutylammoniumbromid, Tetrabutylammoniumiodid, Methyltrioctylammoniumchlorid, Tetraethylphosphoniumbromid und Tetraethylphosphoniumiodid.

11. Verfahren gemäß Anspruch 9, worin der zweite Katalysator ausgewählt ist aus Tetrabutylammoniumhydrogensulfat, Tetrabutylammoniumhalogenid, Tetraethylammoniumhalogenid, Methyltrioctylammoniumchlorid und Tetraethylphosphoniumhalogenid.

12. Verfahren gemäß Anspruch 9, worin die Reaktion des Intermediats in einem Lösungsmittel durchgeführt wird, das ausgewählt ist aus aliphatischen Kohlenwasserstoffen, aromatischen Kohlenwasserstoffen und Chlorkohlenwasserstoffen.

## Revendications

1. Nouveau procédé de fabrication de composés de formule 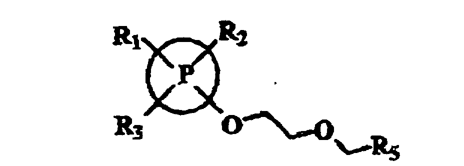 dans laquelle P est choisi parmi phényle et naphtyle ;
dans laquelle R₁, R₂ et R₃ sont choisis chacun indépendamment dans le groupe constitué d'hydrogène, alkyle, alcoxy, aryle, aralkyle, aralcoxy, halogène, alcoxyalcoxy et aralcoxyalcoxy ;
dans laquelle R₅ est choisi parmi phényle substitué ou non substitué et naphtyle substitué ou non substitué, les substituants étant choisis chacun indépendamment parmi alkyle en C₁₋C₈, alcoxy en C₁₋C₈, aroxy, aralcoxy et halogène ;
le procédé comprenant la mise en réaction de phénols ou de naphtols substitués ou non substitués de formule 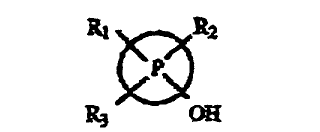 avec du carbonate d'éthylène en présence d'un premier catalyseur choisi dans le groupe constitué d'halogénure de métal, halogénure d'ammonium quaternaire et halogénure de phosphonium quaternaire, formant ainsi un composé intermédiaire de formule 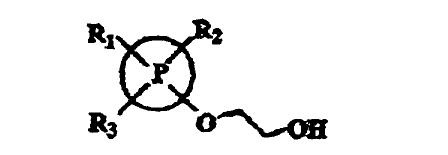 la mise en réaction du composé intermédiaire avec un premier composé choisi dans le groupe constitué d'halogénure d'alkyle, halogénure d'aralkyle, sulfate d'alkyle, sulfate d'aralkyle, sulfonate d'alkyle et sulfonate d'aralkyle, conjointement avec un hydroxyde de métal en présence d'un second catalyseur, le second catalyseur étant choisi parmi sel d'ammonium quaternaire ou sel de phosphonium quaternaire.

2. Procédé selon la revendication 1, dans lequel le premier catalyseur est choisi dans le groupe constitué de chlorure de sodium, bromure de sodium, iodure de sodium, chlorure de potassium, bromure de potassium, iodure de potassium, chlorure de tétraéthylammonium, bromure de tétraéthylammonium, iodure de tétraéthylammonium, chlorure de tétrabutylammonium, bromure de tétrabutylammonium, iodure de tétrabutylammonium, chlorure de méthyltrioctylammonium, bromure de tétraéthylphosphonium et iodure de tétraéthylphosphonium.

3. Procédé selon la revendication 1, dans lequel le second catalyseur est choisi dans le groupe constitué d'hydrogénosulfate de tétrabutylammonium, halogénure de tétrabutylammonium, halogénure de tétraéthylammonium, chlorure de méthyltrioctylammonium et halogénure de tétraéthylphosphonium.

4. Procédé selon la revendication 1, dans lequel on effectue la réaction du composé intermédiaire dans un solvant choisi dans le groupe constitué d'hydrocarbures aliphatiques, hydrocarbures aromatiques et chlorohydro-carbures.

5. Nouveau procédé selon la revendication 1 pour la préparation de composés de formule 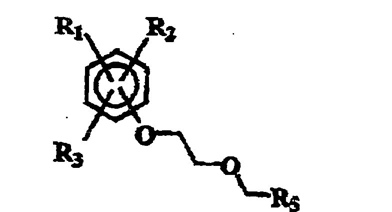 dans laquelle R₁, R₂ et R₃ sont choisis chacun indépendamment dans le groupe constitué d'hydrogène, alkyle, alcoxy, aryle, aralkyle, aralcoxy, halogène, alcoxyalcoxy et aralcoxyalcoxy ;
dans laquelle R₅ est choisi parmi phényle substitué ou non substitué, les substituants étant choisis chacun indépendamment parmi alkyle en C₁₋C₈, alcoxy en C₁₋C₈, aroxy, aralcoxy et halogène ;
le procédé comprenant la mise en réaction de phénols substitués ou non substitués de formule 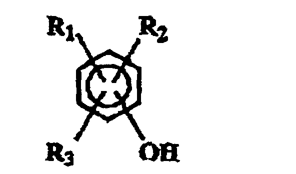 avec du carbonate d'éthylène en présence d'un premier catalyseur choisi dans le groupe constitué d'halogénure de métal, halogénure d'ammonium quaternaire et halogénure de phosphonium quaternaire, formant ainsi un composé intermédiaire de formule 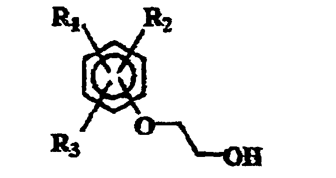 la mise en réaction du composé intermédiaire avec un premier composé choisi dans le groupe constitué d'halogénure d'alkyle, halogénure d'aralkyle, sulfate d'alkyle, sulfate d'aralkyle, sulfonate d'alkyle et sulfonate d'aralkyle, conjointement avec un hydroxyde de métal en présence d'un second catalyseur, le second catalyseur étant choisi parmi sel d'ammonium quaternaire ou sel de phosphonium quaternaire.

6. Procédé selon la revendication 5, dans lequel le premier catalyseur est choisi dans le groupe constitué de chlorure de sodium, bromure de sodium, iodure de sodium, chlorure de potassium, bromure de potassium, iodure de potassium, chlorure de tétraéthylammonium, bromure de tétraéthylammonium, iodure de tétraéthylammonium, chlorure de tétrabutylammonium, bromure de tétrabutylammonium, iodure de tétrabutylammonium, chlorure de méthyltrioctylammonium, bromure de tétraéthylphosphonium et iodure de tétraéthylphosphonium.

7. Procédé selon la revendication 5, dans lequel le second catalyseur est choisi dans le groupe constitué d'hydrogénosulfate de tétrabutylammonium, halogénure de tétrabutylammonium, halogénure de tétraéthylammonium, chlorure de méthyltrioctylammonium et halogénure de tétraéthylphosphonium.

8. Procédé selon la revendication 5, dans lequel on effectue la réaction du composé intermédiaire dans un solvant choisi dans le groupe constitué d'hydrocarbures aliphatiques, hydrocarbures aromatiques et chlorohydro-carbures.

9. Nouveau procédé selon la revendication 1 pour la préparation de composés de formule 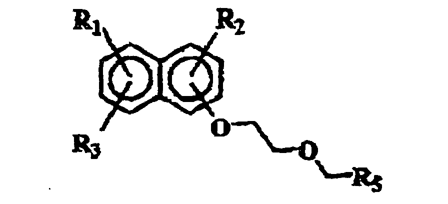 dans laquelle R₁, R₂ et R₃ sont choisis chacun indépendamment dans le groupe constitué d'hydrogène, alkyle, alcoxy, aryle, aralkyle, aralcoxy, halogène, alcoxyalcoxy et aralcoxyalcoxy ;
dans laquelle R₅ est choisi parmi naphtyle substitué ou non substitué, les substituants étant choisis chacun indépendamment parmi alkyle en C₁-C₈, alcoxy en C₁-C₈, aroxy, aralcoxy en C₁-C₈ et halogène ;
le procédé comprenant la mise en réaction de naphtols substitués ou non substitués de formule 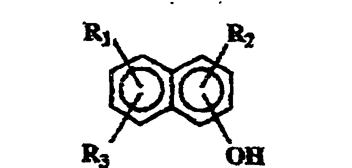 avec du carbonate d'éthylène en présence d'un premier catalyseur choisi dans le groupe constitué d'halogénure de métal, halogénure d'ammonium quaternaire et halogénure de phosphonium quaternaire, formant ainsi un composé intermédiaire de formule 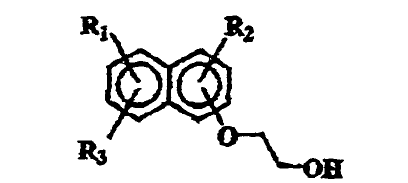 la mise en réaction du composé intermédiaire avec un premier composé choisi dans le groupe constitué d'halogénure d'alkyle, halogénure d'aralkyle, sulfate d'alkyle, sulfate d'aralkyle, sulfonate d'alkyle et sulfonate d'aralkyle, conjointement avec un hydroxyde de métal en présence d'un second catalyseur, le second catalyseur étant choisi parmi sel d'ammonium quaternaire ou sel de phosphonium quaternaire.

10. Procédé selon la revendication 9, dans lequel le premier catalyseur est choisi dans le groupe constitué de chlorure de sodium, bromure de sodium, iodure de sodium, chlorure de potassium, bromure de potassium, iodure de potassium, chlorure de tétraéthylammonium, bromure de tétraéthylammonium, iodure de tétraéthylammonium, chlorure de tétrabutylammonium, bromure de tétrabutylammonium, iodure de tétrabutylammonium, chlorure de méthyltrioctylammonium, bromure de tétraéthylphosphonium et iodure de tétraéthylphosphonium.

11. Procédé selon la revendication 9, dans lequel le second catalyseur est choisi dans le groupe constitué d'hydrogénosulfate de tétrabutylammonium, halogénure de tétrabutylammonium, halogénure de tétraéthylammonium, chlorure de méthyltrioctylammonium et halogénure de tétraéthylphosphonium.

12. Procédé selon la revendication 9, dans lequel on effectue la réaction du composé intermédiaire dans un solvant choisi dans le groupe constitué d'hydrocarbures aliphatiques, hydrocarbures aromatiques et chlorohydro-carbures.
